# EUROPÄISCHE PATENTSCHRIFT

(11) **EP 3 579 811 B1**
(45) Veröffentlichungstag und Bekanntmachung des Hinweises auf die Patenterteilung: **26.05.2021**
(21) Anmeldenummer: 18702659.6
(22) Anmeldetag: 30.01.2018
(51) Int. Cl.: A61K 8/25, A61K 8/29, A61K 8/34, A61K 8/35, A61K 8/37, A61K 8/49, A61Q 17/04, A61Q 19/00, A61K 8/89, A61K 8/92, A61K 8/02

(54) **KOSMETISCHER SONNENSCHUTZSTIFT**
COSMETIC SUNSCREEN STICK
BÂTON COSMETIQUE SUN PROTECTION

(30) Priorität: 08.02.2017 DE 102017201947
(43) Veröffentlichungstag der Anmeldung: 18.12.2019
(73) Patentinhaber: Beiersdorf AG, 20253 Hamburg (DE)
(72) Erfinder: TOSHIHIKO, Shimoda, 5-Chome Chuo-ku 104-0054 Tokyo (JP); WEINERT, Katrin, 22763 Hamburg (DE); BLECKMANN, Andreas, 22926 Ahrensburg (DE)
(86) Internationale Anmeldenummer: PCT/EP2018/052206
(87) Internationale Veröffentlichungsnummer: WO 2018/145943

(56) Entgegenhaltungen:
- WO-A1-2008/009562
- WO-A1-2011/061864
- WO-A2-2010/129321
- DATABASE GNPD [Online] MINTEL; Mai 2015 (2015-05), "Sun Protect Waterproof Lip Balm SPF 30", XP002778799, Database accession no. 3163751
- DATABASE GNPD [Online] MINTEL; Februar 2015 (2015-02), "Sun Protect Lip Balm SPF 30", XP002778800, Database accession no. 2958837
- DATABASE GNPD [Online] MINTEL; September 2010 (2010-09), "Sun Protect Lip Balm SPF 30", XP002778801, Database accession no. 1377572
- DATABASE GNPD [Online] MINTEL; November 2007 (2007-11), "Lip Plump Care", XP002778802, Database accession no. 809244
- DATABASE GNPD [Online] MINTEL; Januar 2013 (2013-01), "Cushiony Essential Gloss", XP002778803, Database accession no. 1971321
- DATABASE GNPD [Online] MINTEL; Juni 2009 (2009-06), "Sun Lip Balm SPF 25", XP002778804, Database accession no. 1119655
- DATABASE GNPD [Online] MINTEL; November 2016 (2016-11), "Gourmand Apricot Fruity Lip Care", XP002778805, Database accession no. 4388573
- DATABASE GNPD [Online] MINTEL; Juni 2013 (2013-06), "Shimmer & Shine Lip Gloss", XP002778806, Database accession no. 2054083
- DATABASE GNPD [Online] MINTEL; März 2006 (2006-03), "Plumping Lip Gloss", XP002778807, Database accession no. 510934
- DATABASE GNPD [Online] MINTEL; März 2012 (2012-03), "Natural Rosy Gloss", XP002778808, Database accession no. 1746598
- DATABASE GNPD [Online] MINTEL; Januar 2017 (2017-01), "Velvet Luminous Matte Lip Colour", XP002778809, Database accession no. 4497029

## Beschreibung

### Beschreibung

### Kosmetischer Sonnenschutzstift

Die vorliegende Erfindung betrifft einen kosmetischen Stift enthaltend ein oder mehrere UV-Filter und ein Fischer-Tropsch-Wachs mit einem Erstarrungspunkt von 80-85 °C (gemessen nach ASTM D 938) und einer Penetration bei 25 °C von 0,4-0,9 mm (gemessen nach ASTM D 1321), wobei die Zubereitung frei ist von 3-(4-Methylbenzyliden)-campher, 2-Hydroxy-4-methoxybenzophenon (INCI: Oxybenzon), 4-Methoxyzimtsäure-2-ethylhexylester (INCI: Octylmethoxycinnamate) und Ethylhexyl-2-cyano-3,3-diphenylacrylat (INCI: Octocrylen), dadurch gekennzeichnet, dass die Zubereitung C20-40 Alkylstearat in einer Konzentration von 0,1 bis 20 Gewichts-%, bezogen auf das Gesamtgewicht der Zubereitung, enthält.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und "UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Eine durchaus schon seit längerem bekannte Produktform stellen die Sonnenschutzstifte wie beispielsweise "Labello sun protect" dar. Diese relativ weichen Stift-Formulierungen mit einer Penetrationshärte bei 25 °C von größer 9 mm sind jedoch nur für den Auftrag auf die Lippen geeignet, während Stiftzubereitungen für die Gesichtshaut allgemein wesentlich härter sein müssen. Auf der anderen Seite kennt der Fachmann eine Vielzahl an UV-Filter haltigen Lotionen, die jedoch so dünnflüssig sind, dass man sie nur mit Applikatoren (z. B. den Fingern) auf die Gesichtshaut auftragen kann. Die WO 2008/009562 A1 beschreibt kosmetische Zubereitung in Stiftform enthaltend a) eine Kombination natürlichen Wachsen, mikrokristallinen Wachsen, synthetischen Wachsen, b) mehreren Ölen c) einem oder mehreren Lipiden. Der Zubereitung können weiter UV-Filter zugesetzt werden. Die Zubereitung enthält aber keine C20-40 Alkylstearate und zeigt keinen Effekt dieser Substanzen auf die Stiftbeschaffenheit oder die Auswirkungen bei der Applikation auf der Haut. Es war daher die Aufgabe einen Sonnenschutzstift zu entwickeln, der sich problemlos auf der Gesichtshaut verteilen lässt. Dieser sollte möglichst eine Penetrationshärte bei 25 °C im Bereich von 4 bis 7 mm aufweisen (gemessen nach DIN 51 579).

Ein weiterer Nachteil an stiftförmigen Sonnenschutzformulierungen besteht in dem Umstand, dass diese üblicherweise 4-Methoxyzimtsäure-2-ethylhexylester (INCI: Octylmethoxycinnamate) und/oder Ethylhexyl-2-cyano-3,3-diphenylacrylat (INCI: Octocrylen) als UV-Filter enthalten. Diese beiden bei Raumtemperatur (25 °C) flüssigen UV-Filter werden üblicherweise benötigt, um die kristallinen UV-Filter 4-(tert.-Butyl)-4'-methoxydiben-zoylmethan, 2,4,6-Tris-[anilino-(p-carbo-2'-ethyl-1'-hexyloxy)]-1,3,5-triazin (INCI: Ethylhexyl Triazone) und 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) in die Zubereitungen "gelöst" einzuarbeiten. Ohne diese weiteren UV-Filter lassen sich keine stiftförmigen Sonnenschutzmittel mit hohen Lichtschutzfaktoren (SPF größer 30, bevorzugt größer/gleich 50) entwickeln und keine Stifte mit einem ausreichend hohen UV-A-Schutz.

4-Methoxyzimtsäure-2-ethylhexylester (INCI: Octylmethoxycinnamate) ist jedoch nicht besonders photostabil, was bei längeren Anwendungszeiten zu einer Reduzierung des UV-Schutzes führen kann. Darüber hinaus besteht die Gefahr, dass sich bei diesem Prozess möglicherweise physiologisch nicht ganz unbedenkliche photochemische Abbauprodukte bilden können.

Der Nachteil am Einsatz von Octocrylen besteht darin, dass trotz der Zulassung durch die Genehmigungsbehörden Octocrylen nicht ganz unumstritten ist und bei Bewertungen bei einigen Verbrauchermagazinen (z.B. Öko-Test) zu "Abwertungen" in der Benotung des Produktes führen. Begründet wird diese Negativ-Bewertung damit, dass einige Wissenschaftler vermuten, dass dieser UV-Filter möglicherweise hormonell wirksam sein könnte. Auch wenn, trotz des jahrzehntelangen weltweiten Einsatzes dieses UV-Filters in Sonnenschutzmitteln keine negativen Wirkungen am Menschen bekannt geworden sind, besteht bei den Verbrauchern der Wunsch, Zubereitungen mit derartigen Inhaltsstoffen zu meiden.

Ferner führen 4-Methoxyzimtsäure-2-ethylhexylester (INCI: Octylmethoxycinnamate) und Ethylhexyl-2-cyano-3,3-diphenylacrylat (INCI: Octocrylen) dazu, dass die Zubereitungen klebrig und fettig-glänzend werden und vom Anwender daher als sensorisch unattraktiv angesehen werden.

Nicht zuletzt besteht bei einer Vielzahl von Sonnenschutzstiften das Problem, dass sie nicht ausreichend kompatibel mit dem für Stifte vorgesehenen Packmittel sind, so dass es zu Stofftransfer aus dem Packmittel in die Zubereitung sowie aus der Zubereitung in das Packmittel kommt.

Es war daher die Aufgabe der vorliegenden Erfindung, eine stiftförmige Sonnenschutzzubereitung ("Sonnenschutzstift") mit hohem Lichtschutzfaktor (SPF größer 30, bevorzugt größer/gleich 50) zu entwickeln, die frei ist von 4-Methoxyzimtsäure-2-ethylhexylester (INCI: Octylmethoxycinnamate) und Ethylhexyl-2-cyano-3,3-diphenylacrylat (INCI: Octocrylen), das zu einem nicht fettig glänzenden sondern eher mattierenden Erscheinungsbild auf der Haut führt, sich leicht und weich auf der Haut verteilen lässt und mit den üblichen Packmitteln für stiftförmige Zubereitungen kompatibel ist.

Überraschend gelöst wird diese Aufgabe durch einen kosmetischen Stift enthaltend
a) ein oder mehrere UV-Filter,
b) ein Fischer-Tropsch-Wachs mit einem Erstarrungspunkt von 80-85 °C (gemessen nach ASTM D 938) und einer Penetration bei 25 °C von 0,4-0,9 mm (gemessen nach ASTM D 1321),
wobei die Zubereitung frei ist von 3-(4-Methylbenzyliden)-campher, 2-Hydroxy-4-methoxybenzophenon (INCI: Oxybenzon), 4-Methoxyzimtsäure-2-ethylhexylester (INCI: Octylmethoxycinnamate) und Ethylhexyl-2-cyano-3,3-diphenylacrylat (INCI: Octocrylen), dadurch gekennzeichnet, dass die Zubereitung C20-40 Alkylstearat in einer Konzentration von 0,1 bis 20 Gewichts-%, bezogen auf das Gesamtgewicht der Zubereitung, enthält.

Überraschend und für den Fachmann nicht vorhersehbar war auch der Umstand, dass die erfindungsgemäßen Stifte, obwohl sie härter sind als die Zubereitungen des Standes der Technik, unter vergleichbaren Auftragungsbedingungen beim Auftragen auf die Haut, deutlich mehr Stift-Substanz auf die Haut übertragen als die Zubereitungen des Standes der Technik. Die höhere und gleichmäßigere Auftragungsmenge lässt sich beispielsweise mit der in der DE 102015203509 A1 offenbarten Methode sichtbar machen.

Es ist erfindungsgemäß vorteilhaft, wenn der erfindungsgemäße Stift dadurch gekennzeichnet ist, dass die Zubereitung das Fischer-Tropsch Wachs in einer Konzentration von mindestens 10 Gewichts-%, bezogen auf das Gesamtgewicht der Zubereitung, enthält.

Erfindungsgemäß bevorzugt ist es, wenn das Fischer-Tropsch Wachs in einer Konzentration von 1 bis 20 Gewichts-%, besonders bevorzugt von 5 bis 20 Gewichts-% und ganz besonders bevorzugt von 7 bis 15%, bezogen auf das Gesamtgewicht der Zubereitung, in der Rezeptur enthalten ist.

Erfindungsgemäß besonders bevorzugt ist das Fischer-Tropsch Wachs mit der CAS-Nummer: 8002-74-2, (EINECS-Nummer 232-315-6), welches die INCI "Synthetic Wax" trägt und bei der Firma Sasol unter dem Handelsnamen "Sasolwax C80" erhältlich ist.

Es ist erfindungsgemäß vorteilhaft, wenn die Zubereitung einen oder mehrere UV-Filter gewählt aus der Gruppe der Verbindungen 4-(tert.-Butyl)-4'-methoxydibenzoylmethan, 2,4,6-Tris-[anilino-(p-carbo-2'-ethyl-1'-hexyloxy)]-1,3,5-triazin (INCI: Ethylhexyl Triazone), 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine), 2-Ethylhexyl 2-hydroxybenzoat (INCI: Ethylhexyl Salicylate) und 3,3,5-Trimethylcyclohexyl 2-hydroxybenzoat (INCI: Homosalate) enthält Die erfindungsgemäßen Ausführungsformen müssen außerdem 0,1 bis 20 Gewichts-% C20-40 Alkylstearat bezogen auf das Gesamtgewicht der Zubereitung enthalten.

Erfindungsgemäß bevorzugte Ausführungsformen der vorliegenden Erfindung sind dadurch gekennzeichnet, dass die Zubereitung 4-(tert.-Butyl)-4'-methoxydibenzoylmethan in einer Konzentration von 0,1 bis 10 Gewichts-%, besonders bevorzugt von 1 bis 10 Gewichts-% und ganz besonders bevorzugt von 3 bis 10 Gewichts-%, bezogen auf das Gesamtgewicht der Zubereitung, enthält.

Ferner sind die erfindungsgemäß bevorzugten Ausführungsformen der vorliegenden Erfindung dadurch gekennzeichnet, dass die Zubereitung 2,4,6-Tris-[anilino-(p-carbo-2'-ethyl-1'-hexyloxy)]-1,3,5-triazin (INCI: Ethylhexyl Triazone) in einer Konzentration von 0,1 bis 5 Gewichts-%, besonders bevorzugt von 0,5 bis 5 Gewichts-% und ganz besonders bevorzugt von 0.5 bis 4 Gewichts-%, bezogen auf das Gesamtgewicht der Zubereitung, enthält.

Außerdem ist es erfindungsgemäß bevorzugt, wenn die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) in einer Konzentration von 0,1 bis 10 Gewichts-%, besonders bevorzugt von 0,1-7 Gewichts-% und ganz besonders bevorzugt von 0.5 bis 7 Gewichts-%, bezogen auf das Gesamtgewicht der Zubereitung, enthält.

Die erfindungsgemäß vorteilhafte Einsatzkonzentration von 2-Ethylhexyl 2-hydroxybenzoat (INCI: Ethylhexyl Salicylate) beträgt von 0,1 bis 10 Gewichts-%, besonders bevorzugt von 1 bis 10 Gewichts-% und ganz besonders bevorzugt von 2 bis10 Gewichts-%, bezogen auf das Gesamtgewicht der Zubereitung, die erfindungsgemäß vorteilhafte Einsatzkonzentration von 3,3,5-Trimethylcyclohexyl 2-hydroxybenzoat (INCI: Homosalate) beträgt von 0 bis 15 Gewichts-%, bezogen auf das Gesamtgewicht der Zubereitung.

Es ist also erfindungsgemäß bevorzugt eine Kombination aus 4-(tert.-Butyl)-4'-methoxydi-benzoylmethan, 2,4,6-Tris-[anilino-(p-carbo-2'-ethyl-1'-hexyloxy)]-1,3,5-triazin (INCI:
Ethylhexyl Triazone), 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine), 2-Ethylhexyl 2-hydroxybenzoat (INCI: Ethylhexyl Salicylate) und 3,3,5-Trimethylcyclohexyl 2-hydroxybenzoat (INCI: Homosalate) einzusetzen.

Erfindungsgemäß vorteilhafte Ausführungsformen der vorliegenden Erfindung sind dadurch gekennzeichnet, dass die Zubereitung Silica und/oder Titandioxid enthält. Dabei ist es erfindungsgemäß bevorzugt, wenn die Zubereitung beide Bestandteile enthält.

Die erfindungsgemäß vorteilhafte Einsatzmenge von Silica Dimethyl Silylate beträgt dabei von 0 bis 7 Gewichts-%, bezogen auf das Gesamtgewicht der Zubereitung.

Die erfindungsgemäß vorteilhafte Einsatzmenge von Titandioxid beträgt dabei von 0 bis 25 Gewichts-%, bezogen auf das Gesamtgewicht der Zubereitung.

Um eine ansprechende stiftförmige Zubereitung zu erhalten ist es erfindungsgemäß von Vorteil, wenn die Zubereitung ein oder mehrere wachsartige Komponenten gewählt aus der Gruppe Cetylpalmitat, Myristylmyristat, Schibutter (INCI: Butyrospermum Parkii Butter) enthält.

Dabei sind erfindungsgemäß bevorzugte Ausführungsformen der vorliegenden Erfindung dadurch gekennzeichnet, dass die Zubereitung Cetylpalmitat in einer Konzentration von 0,1 bis 20 Gewichts-% und bevorzugt von 1 bis 20 Gewichts-%, jeweils bezogen auf das

Gesamtgewicht der Zubereitung, enthält.

Darüber hinaus ist es erfindungsgemäß vorteilhaft, wenn die Zubereitung Myristylmyristat in einer Konzentration von 0,1 bis 20 Gewichts-%, bezogen auf das Gesamtgewicht der Zubereitung, enthält.

Die erfindungsgemäß vorteilhafte Einsatzmenge von Schibutter (INCI: Butyrospermum Parkii Butter) beträgt von 0,1 bis 15 Gewichts-%, bezogen auf das Gesamtgewicht der Zubereitung.

Erfindungsgemäß vorteilhafte Stiftformulierungen sind ferner dadurch gekennzeichnet, dass die Zubereitung Cetylalkohol und/oder Stearylalkohol enthält. Dabei ist die Kombination beider Inhaltsstoffe erfindungsgemäß bevorzugt.

Es ist vorteilhaft im Sinne der vorliegenden Erfindung, wenn die Zubereitung Cetylalkohol in einer Konzentration von 0,1 bis 15 Gewichts-%, bezogen auf das Gesamtgewicht der Zubereitung, enthält.

Außerdem ist es vorteilhaft im Sinne der vorliegenden Erfindung, wenn die Zubereitung Stearylalkohol in einer Konzentration von 0,1 bis 15 Gewichts-%, bezogen auf das Gesamtgewicht der Zubereitung, enthält.

Um eine ansprechende kosmetische Stiftzubereitung zu erhalten, ist es vorteilhaft im Sinne der vorliegenden Erfindung, wenn die Zubereitung eine oder mehrere Ölkomponenten gewählt aus der Gruppe der Verbindungen Di-n-Butyladipat, C12-15 Alkylbenzoat, Butylenglycol dicaprylat/dicaprat (INCI: Butylene Glycol Dicaprylate/Dicaprate), Dimethicon, enthält.

Die erfindungsgemäß bevorzugte Einsatzkonzentration für Di-n-Butyladipat beträgt dabei von 0,1 bis 10 Gewichts-%, bezogen auf das Gesamtgewicht der Zubereitung.

Die erfindungsgemäß bevorzugte Einsatzkonzentration für C12-15 Alkylbenzoat beträgt von 0,1 bis 15 Gewichts-%, bezogen auf das Gesamtgewicht der Zubereitung.

Die erfindungsgemäß bevorzugte Einsatzkonzentration für Butylenglycol dicaprylat/dicaprat (INCI: Butylene Glycol Dicaprylate/Dicaprate) beträgt von 0,1 bis 15 Gewichts-%, bezogen auf das Gesamtgewicht der Zubereitung.

Die erfindungsgemäß bevorzugte Einsatzkonzentration für Dimethicon beträgt dabei von 0,1 bis 20 Gewichts-%, bezogen auf das Gesamtgewicht der Zubereitung.

Die erfindungsgemäße Zubereitung kann erfindungsgemäß vorteilhaft Antioxidantien enthalten, beispielsweise BHT.

Es ist ferner vorteilhaft im Sinne der vorliegenden Erfindung, wenn die erfindungsgemäße Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, ß-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

Erfindungsgemäß vorteilhafte Ausführungsformen der vorliegenden Erfindung sind dadurch gekennzeichnet dass die Zubereitung frei ist von Parabenen, Methylisothiazolinon, Chlormethylisothiazolinon und DMDM-Hydantoin.

### Vergleichsversuch

Die folgenden Vergleichsversuche zeigen mit Beispiel 1 ein Beispiel gemäß der vorliegenden Erfindung. Die Beispiele 2 bis 7 sind Vergleichsbeispiele.

| INCI | Bsp.1 | Bsp.2 | Bsp.3 | Bsp.4 | Bsp.5 | Bsp.6 | Bsp.7 |
|---|---|---|---|---|---|---|---|
| Synthetic Wax | 11,00 | | | | | | |
| C20-40 Alkyl Stearate | 2,00 | 13,00 | | | | | |
| Microcrystalline Wax | | | 11,00 | | | | |
| Carnauba | | | | 11,00 | | | |
| Hydrogenated Castor Oil | | | | | 11,00 | | |
| Myristyl Myristate | 8,00 | 8,00 | 8,00 | 8,00 | 8,00 | 19,00 | 8,00 |
| C12-15 Alkyl Benzoate | 5,00 | 5,00 | 5,00 | 5,00 | 5,00 | 5,00 | 16,00 |
| Dibutyl Adipate | 2,00 | 2,00 | 2,00 | 2,00 | 2,00 | 2,00 | 2,00 |
| Butylene Glycol Dicaprylate/Dicaprate | 3,00 | 3,00 | 3,00 | 3,00 | 3,00 | 3,00 | 3,00 |
| Dimethicone | 10,04 | 10,04 | 10,04 | 10,04 | 10,04 | 10,04 | 10,04 |
| Cetyl Palmitate | 12,00 | 12,00 | 12,00 | 12,00 | 12,00 | 12,00 | 12,00 |
| Butyrospermum Parkii Butter | 8,00 | 8,00 | 8,00 | 8,00 | 8,00 | 8,00 | 8,00 |
| Cetearyl Alcohol | 5,00 | 5,00 | 5,00 | 5,00 | 5,00 | 5,00 | 5,00 |
| Stearyl Alcohol | 5,00 | 5,00 | 5,00 | 5,00 | 5,00 | 5,00 | 5,00 |
| Silica Dimethyl Silylate | 1,00 | 1,00 | 1,00 | 1,00 | 1,00 | 1,00 | 1,00 |
| Perfume | q.s. | q.s. | q.s. | q.s. | q.s. | q.s. | q.s. |
| Homosalate | 9,00 | 9,00 | 9,00 | 9,00 | 9,00 | 9,00 | 9,00 |
| Ethylhexyl Salicylate | 4,75 | 4,75 | 4,75 | 4,75 | 4,75 | 4,75 | 4,75 |
| Bis-Ethylhexyloxyphenol Methoxyphenyl Triazine | 4,00 | 4,00 | 4,00 | 4,00 | 4,00 | 4,00 | 4,00 |
| Ethylhexyl Triazone | 3,50 | 3,50 | 3,50 | 3,50 | 3,50 | 3,50 | 3,50 |
| Butyl Methoxydibenzoylmethane | 4,75 | 4,75 | 4,75 | 4,75 | 4,75 | 4,75 | 4,75 |
| Titanium Dioxide (nano) | 0,82 | 0,82 | 0,82 | 0,82 | 0,82 | 0,82 | 0,82 |
| Silica | 0,15 | 0,15 | 0,15 | 0,15 | 0,15 | 0,15 | 0,15 |
| - - - - - - - | | | | | | | |
| | Basis 1 | | | | | | |
| Texture vs Basis 1 | | Oily | Oily | Sticky | Sticky | Oily and Sticky | Oily and Sticky |
| Penetration (mm) | 5,8 | 7.4 | 9,4 | 7,6 | 7.1 | 13 | 15,3 |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **INCI** | **Bsp.8** | **Bsp.9** | **Bsp.10** | **Bsp.11** | **Bsp.12** | **Bsp.13** |
|---|---|---|---|---|---|---|
| Synthetic Wax | 11,00 | 11,00 | 11,00 | 11,00 | 11,00 | 9,00 |
| C20-40 Alkyl Stearate | 2,00 | 2,00 | 2,00 | 2,00 | 2,00 | 5,00 |
| Myristyl Myristate | 12,00 | 8,00 | 15,00 | 8,00 | 5,00 | 8,00 |
| C12-15 Alkyl Benzoate | 10,00 | 5,00 | 5,00 | 5,00 | 5,00 | 5,00 |
| Dibutyl Adipate | 7,00 | 7,00 | 2,00 | 2,00 | 2,00 | 1,00 |
| Butylene Glycol Dicaprylate/Dicaprate | 7,00 | 3,00 | 3,00 | 3,00 | 3,00 | 1,00 |
| Dimethicone | ad 100 | ad 100 | ad 100 | ad 100 | ad 100 | ad 100 |
| Cetyl Palmitate | 12,00 | 12,00 | 5,00 | 15,00 | 12,00 | 12,00 |
| Butyrospermum Parkii Butter | 8,00 | 8,00 | 8,00 | 8,00 | 8,00 | 8,00 |
| Cetearyl Alcohol | 5,00 | 5,00 | 5,00 | 5,00 | 5,00 | 5,00 |
| Stearyl Alcohol | 5,00 | 5,00 | 5,00 | 5,00 | 5,00 | 5,00 |
| Silica Dimethyl Silylate | 0,00 | 5,00 | 1,00 | 1,00 | 1,00 | 1,00 |
| Perfume | q.s. | q.s. | q.s. | q.s. | q.s. | q.s. |
| Homosalate | 0,00 | 0,00 | 9,00 | 9,00 | 9,00 | 9,00 |
| Ethylhexyl Salicylate | 3,00 | 4,50 | 4,75 | 4,75 | 4,75 | 4,75 |
| Bis-Ethylhexyloxyphenol Methoxyphenyl Triazine | 1,00 | 3,00 | 4,00 | 4,00 | 4,00 | 4,00 |
| Ethylhexyl Triazone | 0,50 | 2,00 | 3,50 | 3,50 | 3,50 | 3,50 |
| Butyl Methoxydibenzoylmethane | 3,00 | 3,50 | 4,75 | 4,75 | 4,75 | 4,75 |
| Titanium Dioxide (nano) | 0,00 | 0,00 | 0,82 | 0,82 | 0,82 | 0,82 |
| Silica | 0,00 | 0,00 | 0,15 | 0,15 | 0,15 | 0,15 |

## Patentansprüche

1. Kosmetischer Stift enthaltend
a) ein oder mehrere UV-Filter,
b) ein Fischer-Tropsch-Wachs mit einem Erstarrungspunkt von 80-85 °C (gemessen nach ASTM D 938) und einer Penetration bei 25 °C von 0,4-0,9 mm (gemessen nach ASTM D 1321),
wobei die Zubereitung frei ist von 3-(4-Methylbenzyliden)-campher, 2-Hydroxy-4-methoxybenzophenon (INCI: Oxybenzon), 4-Methoxyzimtsäure-2-ethylhexylester (INCI: Octylmethoxycinnamate) und Ethylhexyl-2-cyano-3,3-diphenylacrylat (INCI: Octocrylen), **dadurch gekennzeichnet, dass** die Zubereitung C20-40 Alkylstearat in einer Konzentration von 0,1 bis 20 Gewichts-%, bezogen auf das Gesamtgewicht der Zubereitung, enthält.

2. Kosmetischer Stift nach Anspruch 1 **dadurch gekennzeichnet, dass** die Zubereitung das Fischer-Tropsch Wachs in einer Konzentration von 1 bis 20 Gewichts-%, bezogen auf das Gesamtgewicht der Zubereitung, enthält.

3. Kosmetischer Stift nach einem der vorhergehenden Ansprüche, **dadurch gekennzeichnet, dass** die Zubereitung einen oder mehrere UV-Filter gewählt aus der Gruppe der Verbindungen 4-(tert.-Butyl)-4'-methoxydibenzoylmethan, 2,4,6-Tris-[anilino-(p-carbo-2'-ethyl-1'-hexyloxy)]-1,3,5-triazin (INCI: Ethylhexyl Triazone), 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine), 2-Ethylhexyl 2-hydroxybenzoat (INCI: Ethylhexyl Salicylate) und 3,3,5-Trimethylcyclohexyl 2-hydroxybenzoat (INCI: Homosalate) enthält.

4. Kosmetischer Stift nach einem der vorhergehenden Ansprüche, **dadurch gekennzeichnet, dass** die Zubereitung 4-(tert.-Butyl)-4'-methoxydibenzoylmethan in einer Konzentration von 0,1 bis 10 Gewichts-%, bezogen auf das Gesamtgewicht der Zubereitung, enthält.

5. Kosmetischer Stift nach einem der vorhergehenden Ansprüche, **dadurch gekennzeichnet, dass** die Zubereitung 2,4,6-Tris-[anilino-(p-carbo-2'-ethyl-1'-hexyl-oxy)]-1,3,5-triazin (INCI: Ethylhexyl Triazone) in einer Konzentration von 0,1 bis 5 Gewichts-%, bezogen auf das Gesamtgewicht der Zubereitung, enthält.

6. Kosmetischer Stift nach einem der vorhergehenden Ansprüche, **dadurch gekennzeichnet, dass** die Zubereitung 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) in einer Konzentration von 0,1 bis 10 Gewichts-%, bezogen auf das Gesamtgewicht der Zubereitung, enthält.

7. Kosmetischer Stift nach einem der vorhergehenden Ansprüche, **dadurch gekennzeichnet, dass** die Zubereitung Silica und/oder Titandioxid enthält.

8. Kosmetischer Stift nach einen der vorhergehenden Ansprüche, **dadurch gekennzeichnet, dass** die Zubereitung ein oder mehrere wachsartige Komponenten gewählt aus der Gruppe Cetylpalmitat, Myristylmyristat, Schibutter (INCI: Butyrospermum Parkii Butter) enthält.

9. Kosmetischer Stift nach einem der vorhergehenden Ansprüche, **dadurch gekennzeichnet, dass** die Zubereitung Cetylpalmitat in einer Konzentration von 0,1-bis 20 Gewichts-%, bezogen auf das Gesamtgewicht der Zubereitung, enthält.

10. Kosmetischer Stift nach einem der vorhergehenden Ansprüche, **dadurch gekennzeichnet, dass** die Zubereitung Myristylmyristat in einer Konzentration von 0,1 bis 20 Gewichts-%, bezogen auf das Gesamtgewicht der Zubereitung, enthält.

11. Kosmetischer Stift nach einem der vorhergehenden Ansprüche, **dadurch gekennzeichnet, dass** die Zubereitung Cetylalkohol und/oder Stearylalkohol enthält.

12. Kosmetischer Stift nach einem der vorhergehenden Ansprüche, **dadurch gekennzeichnet, dass** die Zubereitung eine oder mehrere Ölkomponenten gewählt aus der Gruppe der Verbindungen Di-n-Butyladipat, C12-15 Alkylbenzoat, Butylenglycol dicaprylat/dicaprat (INCI: Butylene Glycol Dicaprylate/Dicaprate), Dimethicon, enthält.

## Claims

1. Cosmetic stick comprising
a) one or more UV filters,
b) a Fischer-Tropsch wax having a solidification point of 80-85°C (measured according to ASTM D 938) and a penetration at 25°C of 0.4-0.9 mm (measured according to ASTM D 1321),
with the preparation being free of 3-(4-methylbenzylidene)camphor, 2-hydroxy-4-methoxybenzophenone (INCI: Oxybenzone), 2-ethylhexyl 4-methoxycinnamate (INCI: Octyl Methoxycinnamate) and ethylhexyl 2-cyano-3,3-diphenylacrylate (INCI: Octocrylene), **characterized in that** the preparation contains C20-C40 alkyl stearate in a concentration of 0.1% to 20% by weight based on the total weight of the preparation.

2. Cosmetic stick according to Claim 1, **characterized in that** the preparation contains the Fischer-Tropsch wax in a concentration from 1% to 20% by weight based on the total weight of the preparation.

3. Cosmetic stick according to either of the preceding claims, **characterized in that** the preparation comprises one or more UV filters selected from the group of compounds 4-(tert-butyl)-4'-methoxydibenzoylmethane, 2,4,6-tris[anilino(p-carbo-2'-ethyl-1'-hexyloxy)]-1,3,5-triazine (INCI: Ethylhexyl Triazone), 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine), 2-ethylhexyl 2-hydroxybenzoate (INCI: Ethylhexyl Salicylate) and 3,3,5-trimethylcyclohexyl 2-hydroxybenzoate (INCI: Homosalate).

4. Cosmetic stick according to any of the preceding claims, **characterized in that** the preparation contains 4-(tert-butyl)-4'-methoxydibenzoylmethane in a concentration from 0.1% to 10% by weight based on the total weight of the preparation.

5. Cosmetic stick according to any of the preceding claims, **characterized in that** the preparation contains 2,4,6-tris[anilino(p-carbo-2'-ethyl-1'-hexyloxy)]-1,3,5-triazine (INCI: Ethylhexyl Triazone) in a concentration from 0.1% to 5% by weight based on the total weight of the preparation.

6. Cosmetic stick according to any of the preceding claims, **characterized in that** the preparation contains 2,4-bis{[4-(2-ethylhexyloxy)-2-hydroxy]-phenyl}-6-(4-methoxyphenyl)-1,3,5-triazine (INCI: Bis-Ethylhexyloxyphenol Methoxyphenyl Triazine) in a concentration from 0.1% to 10% by weight based on the total weight of the preparation.

7. Cosmetic stick according to any of the preceding claims, **characterized in that** the preparation comprises silica and/or titanium dioxide.

8. Cosmetic stick according to any of the preceding claims, **characterized in that** the preparation comprises one or more waxy constituents selected from the group cetyl palmitate, myristyl myristate, shea butter (INCI: Butyrospermum Parkii Butter).

9. Cosmetic stick according to any of the preceding claims, **characterized in that** the preparation contains cetyl palmitate in a concentration from 0.1% to 20% by weight based on the total weight of the preparation.

10. Cosmetic stick according to any of the preceding claims, **characterized in that** the preparation contains myristyl myristate in a concentration from 0.1% to 20% by weight based on the total weight of the preparation.

11. Cosmetic stick according to any of the preceding claims, **characterized in that** the preparation comprises cetyl alcohol and/or stearyl alcohol.

12. Cosmetic stick according to any of the preceding claims, **characterized in that** the preparation comprises one or more oily constituents selected from the group of compounds di-n-butyl adipate, C12-15 alkyl benzoate, butylene glycol dicaprylate/dicaprate (INCI: Butylene Glycol Dicaprylate/Dicaprate), dimethicone.

## Revendications

1. Bâton cosmétique contenant
a) un ou plusieurs filtres UV,
b) une cire de type Fischer-Tropsch dotée d'un point de solidification de 80 à 85 °C (mesuré d'après la norme ASTM D 938) et d'une pénétration à 25 °C de 0,4 à 0,9 mm (mesurée d'après la norme ASTM D 1321),
la préparation étant exempte de 3-(4-méthylbenzylidène)-camphre, de 2-hydroxy-4-méthoxybenzophénone (INCI : Oxybenzon), d'ester de 2-éthylhexyle d'acide 4-méthoxycinnamique (INCI : Octylmethoxycinnamate) et de 2-cyano-3,3-diphénylacrylate d'éthylhexyle (INCI : Octocrylen), **caractérisé en ce que** la préparation contient un stéarate de C20-40 alkyle en une concentration de 0,1 à 20 % en poids, par rapport au poids total de la préparation.

2. Bâton cosmétique selon la revendication 1 **caractérisé en ce que** la préparation contient une cire de type Fischer-Tropsch en une concentration de 1 à 20 % en poids, par rapport au poids total de la préparation.

3. Bâton cosmétique selon l'une quelconque des revendications précédentes, **caractérisé en ce que** la préparation contient un ou plusieurs filtres UV choisis dans le groupe des composés 4-(tert.-butyl)-4'-méthoxydibenzoylméthane, 2,4,6-tris- [anilino-(p-carbo-2'-éthyl-1'-hexyloxy)]-1,3,5-triazine (INCI : Ethylhexyl Triazone), 2,4-bis-{[4-(2-éthyl-hexyloxy)-2-hydroxy]-phényl}-6-(4-méthoxyphényl)-1,3,5-triazine (INCI : Bis- Ethylhexyloxyphenol methoxyphenyl Triazine), 2-hydroxybenzoate de 2-éthylhexyle (INCI: Ethylhexyl Salicylate) et 2-hydroxybenzoate de 3,3,5-triméthylcyclohexyle (INCI : Homosalate).

4. Bâton cosmétique selon l'une quelconque des revendications précédentes, **caractérisé en ce que** la préparation contient du 4-(tert.-butyl)-4'-méthoxydibenzoylméthane en une concentration de 0,1 à 10 % en poids, par rapport au poids total de la préparation.

5. Bâton cosmétique selon l'une quelconque des revendications précédentes, **caractérisé en ce que** la préparation contient de la 2,4,6-tris- [anilino-(p-carbo-2'-éthyl-1'-hexyloxy)]-1,3,5-triazine (INCI : Ethylhexyl Triazone) en une concentration de 0,1 à 5 % en poids, par rapport au poids total de la préparation.

6. Bâton cosmétique selon l'une quélconque des revendications précédentes, **caractérisé en ce que** la préparation contient de la 2,4-bis-{[4-(2-éthyl-hexyloxy)-2-hydroxy]-phényl}-6-(4-méthoxyphényl)-1,3,5-triazine (INCI : Bis- Ethylhexyloxyphenol methoxyphenyl Triazine) en une concentration de 0, 1 à 10 % en poids, par rapport au poids total de la préparation.

7. Bâton cosmétique selon l'une quelconque des revendications précédentes, **caractérisé en ce que** la préparation contient une silice et/ou un dioxyde de titane.

8. Bâton cosmétique selon l'une quelconque des revendications précédentes, **caractérisé en ce que** la préparation contient un ou plusieurs composants de type cire choisis dans le groupe du palmitate de cétyle, du myristate de myristyle, du beurre de karité (INCI: Butyrospermum Parkii Butter).

9. Bâton cosmétique selon l'une quelconque des revendications précédentes, **caractérisé en ce que** la préparation contient du palmitate de cétyle en une concentration de 0,1 à 20 % en poids, par rapport au poids total de la préparation.

10. Bâton cosmétique selon l'une quelconque des revendications précédentes, **caractérisé en ce que** la préparation contient du myristate de myristyle en une concentration de 0,1 à 20 % en poids, par rapport au poids total de la préparation.

11. Bâton cosmétique selon l'une quelconque des revendications précédentes, **caractérisé en ce que** la préparation contient de l'alcool cétylique et/ou de l'alcool stéarique.

12. Bâton cosmétique selon l'une quelconque des revendications précédentes, **caractérisé en ce que** la préparation contient un ou plusieurs composants de type huile choisis dans le groupe des composés adipate de di-n-butyle, benzoate de C12-15 alkyle, dicaprylate/dicaprate de butylèneglycol (INCI : Butylene Glycol Dicaprylate/Dicaprate), diméthicone.
